# EUROPÄISCHE PATENTANMELDUNG

(11) **EP 2 455 344 A1**
(43) Veröffentlichungstag der Anmeldung: **23.05.2012**
(21) Anmeldenummer: 10014854.3
(22) Anmeldetag: 23.11.2010
(51) Int. Cl.: C02F 1/461

(54) **Elektrolysezelle sowie Anlage und Verfahren zur Erzeugung einer elektro-chemisch aktivierten Lösung durch Elektrolyse**

(71) Anmelder: Caliopa AG, 6314 Unterägeri (CH)
(72) Erfinder: Mathé, Hans-Georg, 6330 Cham (CH)
(74) Vertreter: Tergau & Walkenhorst

(57) **Zusammenfassung**

Eine Elektrolysezelle (40), insbesondere zur Erzeugung einer elektrochemisch aktivierten Kochsalzlösung, mit einem mit einer Anode versehenen Anodenraum (44) und mit einem von diesem durch eine Membran (46) getrennten, mit einer Kathode versehenen Kathodenraum (42), soll auf besonders günstige Weise die Herstellung einer elektrochemisch aktivierten Salzlösung ermöglichen, für die bei hoher Lagerbeständigkeit eine besonders hohe bakteriozide oder antibakterielle Wirkung, charakterisiert durch einen hohen Gehalt an freiem Chlor, gewährleistet ist, wobei der pH-Wert möglichst eine gute Vereinbarkeit mit anderen Substanzen oder Wirkstoffen erlauben oder sogar in der Art eines freien Parameters unabhängig von den anderen genannten Parametern einstellbar gehalten werden sollte. Dazu weist die Anode erfindungsgemäß auf ihrer Oberfläche eine Beschichtung (92) umfassend Rutheniumoxid und Iridiumoxid auf. In einer Anlage (1) zur Erzeugung einer elektrochemisch aktivierten Lösung mit einem eine Mehrzahl von derartigen Elektrolysezellen (40, 41) und die Anodenräume (44) der Elektrolysezellen (40, 41) medienseitig in Reihe geschaltet sind, wobei die Oberflächenbeschichtung (92) der Anode jeder Elektrolysezelle (40 ,41) jeweils abhängig von der Position der jeweiligen Elektrolysezelle (40, 41) in der Reihenschaltung gewählt ist.

## Beschreibung

Die Erfindung betrifft eine Elektrolysezelle, insbesondere zur Erzeugung einer elektrochemisch aktivierten Kochsalzlösung, mit einem mit einer Anode versehenen Anodenraum und mit einem von diesem durch eine Membran getrennten, mit einer Kathode versehenen Kathodenraum. Sie bezieht sich weiter auf eine Anlage zur Erzeugung einer elektrochemisch aktivierten Lösung mit einem eine Mehrzahl von derartigen Elektrolysezellen aufweisenden Elektrolysemodell sowie auf ein Verfahren zur Erzeugung einer elektrochemisch aktivierten Lösung durch Elektrolyse von solehaltigem Wasser unter Verwendung einer derartigen Anlage.

Elektrolysevorrichtungen oder -anlagen zur Erzeugung einer elektrochemisch aktivierten Lösung durch Elektrolyse von solehaltigem Wasser, bei denen eine oder mehrerer Elektrolysezellen der oben genannten Art zum Einsatz kommen, sind aus einer Vielzahl von Dokumenten, beispielsweise aus der DE 30 003 131 A1, aus der US 4,056,452, aus der EP 1 728 768 A1 oder aus der nicht vorveröffentlichten europäischen Patentanmeldung Nr. 10 003 555.9-2104 bekannt. In derartigen Anlagen wird zur Erzeugung der elektrochemisch aktivierten Salzlösung durch Elektrolyse ein mit einer Kochsalzlösung oder mit Sole beaufschlagter Wasserstrom der Elektrolysevorrichtung zugeführt und dort elektrolytisch zersetzt. Dadurch wird eine elektrochemisch aktivierte wässrige Salzlösung erhalten, die einen vergleichsweise hohen Gehalt an freiem Chlor und ein vergleichsweise hohes Redox-Potential aufweist. Die dabei erhältliche elektrochemisch aktivierte Salzlösung ist besonders günstig als Desinfektionsmittel, beispielsweise zur Entkeimung von Wasser und/oder wässrigen Lösungen, nutzbar.

Bei der Herstellung der elektrochemisch aktivierten Salzlösung auf die genannte Weise wird üblicherweise eine besonders hohe Wirksamkeit oder Biozidität der hergestellten Substanz, üblicherweise charakterisiert durch einen besonders hohen Gehalt an freiem Chlor und/oder ein angemessen hohes Redox-Potential, angestrebt. Zudem ist gerade im Hinblick auf mögliche Anwendungen derartiger Substanzen als Desinfektionsmittel oder auch als Zusatz-, Träger- oder sogar Wirkstoff in medizinischen oder therapeutischen Präparaten wünschenswert, dass die hohe Wirksamkeit gerade im Hinblick auf eine besonders gute Lagerbeständigkeit auch nach vergleichsweise langer Lagerung von beispielsweise mehr als einem Jahr nahezu unverändert oder mit nur geringfügigen Veränderungen aufrecht erhalten bleiben soll. Ein weiterer wichtiger Kennwert für die Charakterisierung der auf die genannte Weise erhältlichen elektrochemisch aktivierten Salzlösung ist deren pH-Wert, der die Kompatibilität und Vereinbarkeit mit anderen chemischen Substanzen oder Wirkstoffen und auch die Verwendbarkeit der elektrochemisch aktivierten Salzlösungen in verschiedenen Umgebungsbedingungen und der Gleichen wesentlich mitbestimmt.

Bei der Herstellung von elektrochemisch aktivierten Salzlösungen der genannten Art durch Elektrolyse besteht somit allgemein das Bestreben, bei hoher Lagerbeständigkeit eine besonders hohe bakteriozide oder antibakterielle Wirkung, charakterisiert durch einen hohen Gehalt an freiem Chlor, zu gewährleisten, wobei der pH-Wert möglichst eine gute Vereinbarkeit mit anderen Substanzen oder Wirkstoffen erlauben oder sogar in der Art eines freien Parameters unabhängig von den anderen genannten Parametern einstellbar gehalten werden sollte. Gerade im Hinblick auf diese Parameter und auf ihre Vereinbarkeit miteinander sind die bekannten Konzepte zur Herstellung der elektrochemisch aktivierten Salzlösung jedoch nur begrenzt einsetzbar.

Der Erfindung liegt daher die Aufgabe zugrunde, eine Elektrolysezelle der oben genannten Art anzugeben, mit der auf besonders günstige Weise eine elektrochemisch aktivierte Salzlösung herstellbar ist, die die genannten Anforderungen in besonders weitgehendem Maße erfüllt. Des Weiteren sollen eine für diesen Einsatzzweck besonders geeignete Anlage mit einer Mehrzahl derartiger Elektrolysezellen sowie ein Verfahren zur Erzeugung einer elektrochemisch aktivierten Lösung durch Elektrolyse mit den genannten Eigenschaften angegeben werden. Bezüglich der Elektrolysezelle wird diese Aufgabe erfindungsgemäß dadurch gelöst, dass die Anode auf ihrer Oberfläche eine Beschichtung umfassend Rutheniumoxid, insbesondere RuO₂ und/oder RuO₄, und Iridiumoxid, insbesondere IrO₂ und/oder IrO₃, aufweist.

Die Erfindung geht dabei von der Überlegung aus, dass die Elektrolysezelle der oben genannten Art für die Herstellung einer elektrochemisch aktivierten Salzlösung mit besonders hoher bakteriozider oder antibakterieller Wirkung bereitgestellt werden kann, indem sie in ihrer Gesamtheit in besonderem Maße auf eine besonders effiziente und wirksame Anreicherung des Aktivchlorgehalts in der Salzlösung in der Art eines primären Auslegungsziels ausgelegt ist. Dabei liegt insbesondere die Erkenntnis zugrunde, dass der Aktivchlorgehalt in der hergestellten Salzlösung als Sauerstoff-abspaltende Verbindung bei einem Kontakt mit anderen Materialien wirkt; der Aktivchlorgehalt kann somit als Maß für die Aktivität und Wirksamkeit der hergestellten Salzlösung hinsichtlich der Sauerstoff-Anspaltung in anderen Materialien angesehen werden. Die auf den Aktivchlorgehalt bezogenen Ausführungen sind somit sinngemäß und ggf. unter Anpassung materialspezifischer Angaben auch auf andere Sauerstoff-abspaltende Verbindungen übertragbar.

Die Anreicherung der Salzlösung mit Chlor erfolgt dabei bauartbedingt und im Hinblick auf die Ionenwanderung durch die den Anodenraum einerseits und den Kathodenraum andererseits der Elektrolysezelle voneinander trennende Membran hindurch im Anodenraum, so dass dieser in besonderem Maße für eine gezielte und wirksame Bereitstellung von Chlor hin ausgelegt sein sollte. Um dabei die elektrochemische Umwandlung der über die Membran in den Anodenraum eintretenden Cl⁻-Ionen in stabile, eine vergleichsweise lange Haltbarkeit der entstehenden Substanz begünstigende Verbindungsformen zu ermöglichen oder zu erleichtern, sollte daher im Anodenraum gezielt eine entsprechende Umsetzung der eintretenden Cl⁻-Ionen unterstützt werden.

Wie sich überraschend herausgestellt hat, ist dies in besonders günstigem Maße erreichbar, indem an einem geeigneten Ort der Umsetzung der Cl⁻-Ionen - oder ggf. auch von O₂⁻-Ionen - in der Art einer katalytischen Aktivierung geeignet in den Umwandlungsprozess eingegriffen wird. Aufgrund der elektrochemischen Umwandlungsprozesse hat sich hierbei die Anode selbst und ihre Oberfläche als besonders geeigneter Ort für die Bereitstellung eines geeigneten katalytisch wirksamen Stoffs erwiesen. Die Anode sollte daher an ihrer Oberfläche mit einer geeigneten Beschichtung versehen sein, die hinsichtlich ihrer Zusammensetzung gezielt auf die Förderung der Transformationsprozesse bei der Entstehung der Chlor-haltigen Verbindungen bzw. ggf. auch anderer Sauerstoff-abspaltender Verbindungen in der elektrochemisch aktivierten Salzlösung im Anodenraum ausgerichtet ist. Wie sich überraschender Weise herausgestellt hat, sind gerade die Bestandteile Rutheniumoxid und/oder Iridiumoxid in der Oberflächenbeschichtung für die Anode hierfür besonders geeignet.

Die Oberflächen-Beschichtung der Anode kann dabei durch gängige Beschichtungsverfahren aufgebracht werden. Um aber gerade im Hinblick auf die gewünschten katalytischen Eigenschaften bei der Umsetzung der Chlor-Verbindungen bzw. auch anderer Sauerstoff-abspaltender Verbindungen eine besonders homogene Aktivität und damit insgesamt einen besonders hohen Wirkungsgrad sicherzustellen, ist die Oberflächenbeschichtung in besonders vorteilhafter Ausgestaltung erhalten durch Bestreichen eines für die Bildung der Anode vorgesehenen Trägerkörpers mit einer die Metallanteile enthaltenden Paste und anschließendes Brennen und Oxidieren.

Vorteilhafterweise wird dabei der Trägerkörper mit der die Metallanteile enthaltenden Paste in mehrfach aufeinander folgenden Bearbeitungsschritten jeweils dünn bestrichen, wobei die Paste nachfolgend zum Ausbrennen und Oxidieren der Metallanteile einem thermischen Behandlungsprozess, vorzugsweise unter Sauerstoff-Angebot oder Sauerstoff-Überschuss, ausgesetzt wird. Diese Teilschritte, also das Bestreichen mit der Paste und das anschließende Ausbrennen und Oxidieren, kann in mehreren Zyklen wiederholt angewendet werden, so dass sukszessive die Oberflächenbeschichtung aufgebaut wird. Hierdurch sind vergleichsweise hohe Beschichtungs-Dicken bei hoher Homogenität der Beschichtung insgesamt erreichbar. Beim Auftragen kann dabei die Paste besonders bevorzugt in Form einer metallhaltigen Lösung oder dergleichen vorgehalten werden, die auf den für die Anode vorgesehenen Trägerkörper geeignet aufgebracht oder aufgestrichen wird.

Gerade durch die Verwendung der beiden genannten Bestandteile, nämlich Rutheniumoxid einerseits und Iridiumoxid andererseits, in der Oberflächenbeschichtung für die Anode werden mehrere Auslegungsziele in besonders günstiger Weise kombiniert erreicht. Der Bestandteil Rutheniumoxid in der Beschichtung ist dabei in erster Linie als günstiger Katalysator für eine besonders hohe Produktionsrate an Sauerstoff-abspaltenden Substanzen, insbesondere gemessen in freiem Chlor, vorgesehen. Der weitere Bestandteil Iridiumoxid hingegen begünstigt in besonderem Maße die Stabilität der Anode gegen Abnutzung durch den Elektrolyseprozess, wobei sich zudem überraschender Weise herausgestellt hat, dass durch den Bestandteil Iridiumoxid in der Oberflächenbeschichtung auch die Langzeitstabilität der hergestellten elektrochemisch aktivierten Salzlösung besonders begünstigt werden kann (wie gesagt, nur als nicht nachweisbare Behauptung, was aber gut sein könnte, da sowieso stabil). Grundsätzlich wurde beobachtet, dass mit höherem Ru-Anteil in der Oberflächenbeschichtung ein geringerer Stromfluss in der Elektrolysezelle erforderlich ist, um eine vorgegebene Produktmenge an elektrochemisch aktivierter Salzlösung herzustellen. Damit ist mit erhöhtem Ru-Anteil in der Oberflächenbeschichtung auch eine geringere Wärmeentwicklung beim Elektrolyseprozess in Kauf zu nehmen, so dass die - infolge der Wärmeentwicklung auftretende - unerwünschte Produktion von Nebenprodukten wie beispielsweise Chloraten vergleichsweise geringer gehalten werden kann.

Im Hinblick auf die Mischungsverhältnisse bei der Herstellung oder Bildung der Beschichtung ist daher vorzugsweise ein besonders bedarfsgerecht angepasstes Anteilsverhältnis von Rutheniumoxid einerseits (begünstigt eine hohe Cl-Produktionsrate) und Iridiumoxid andererseits (begünstigt Sauerstoff oder O₂-Produktion und auch die Langzeitstabilität des Produkts bei einem pH-Wert von vorzugsweise etwa 7,4) vorgesehen. Um dies auf besonders günstige Weise zu gewährleisten, sind die Bestandteile der Oberflächenbeschichtung der Anode in besonders vorteilhafter Ausgestaltung in einem Verhältnis Rutheniumoxid/Iridiumoxid, insbesondere die Gewichtsanteile in der zum Bestreichen des Trägerkörpers der Anode vorgesehenen Ausgangspaste, von zwischen 50/50 (Ru/Ir, dieses Verhältnis begünstigt besonders die Stabilität der Anode) und 95/5 (Ru/Ir, dieses Verhältnis begünstigt besonders die Stoffproduktion), besonders bevorzugt von etwa 70/30 (Ru/Ir), vorhanden. Die angegeben Verhältnisse bezeichnen dabei insbesondere die jeweiligen Mischungsverhältnisse vor dem Auftragen.

Bei der Herstellung der Beschichtung werden die verfahrensrelevanten Parameter wie insbesondere Behandlungstemperaturen, Umgebungsbedingungen und Behandlungsdauern bei der Herstellung, Aufbringung und/oder thermischen Nachbehandlung der Ausgangspaste, vorzugsweise entsprechend dem vorgesehenen Mischungsverhältnis und für dieses geeignet gewählt. Hinsichtlich des Rutheniumoxids wird dabei vorteilhafterweise eine möglichst weitgehende Umsetzung zu Ruthenium (IV)-Oxid (RuO₂) angestrebt, das eine Dichte von etwa 6,97 g/cm³ aufweist. Hingegen sollten möglichst wenig Ruthenium (VIII)-Oxid (RuO₄) und auch möglichst wenig RuO₃ zurückbleiben. Um dies zu gewährleisten, werden vorzugsweise bei der thermischen Nachbehandlung der aufgetragenen Beschichtung vergleichsweise hohe Temperaturen von insbesondere mehr als 800°C und zudem eine Atomsphäre mit Sauerstoffüberschuss verwendet, um eine möglichst vollständige Umsetzung der rutheniumhaltigen Bestandteile zu RuO₂ zu gewährleisten. Bei Anwesenheit des Iridiums wird dieses beim Brennen in O₂-Atmosphäre zu IrO₂ umgesetzt, was wiederum die Festigkeit und Beständigkeit des Gesamtsystems weiter begünstigt.

Hinsichtlich ihrer weiteren charakteristischen Parameter, insbesondere ihrer Schichtdicke, ist die Oberflächenbeschichtung der Anode vorteilhafterweise gezielt an die Erfordernisse bei der katalytischen Unterstützung der Cl-Produktion und/oder der O₂-Produktion in Rahmen des Elektrolysevorgangs angepasst. Im Hinblick auf die weiteren Geometrieparameter wie beispielsweise die aktive Oberfläche und sonstige Bauteilgrößen weist die Oberflächenbeschichtung der Anode vorteilhafterweise eine mittlere Schichtdicke auf die einer Flächendichte des Edelmetallanteils von etwa 5 g/m² bis etwa 11 g/m² entspricht. Vorzugsweise ist dabei für eine Beschichtung des Typs "Ru-Mischoxid", entsprechend einem Verhältnis Ru/Ir von 95/5, eine Flächendichte des Edelmetalls von 5 g/m², für eine Beschichtung Typs "Ru-IR Mischoxid" in einer ersten Variante, entsprechend einem Verhältnis Ru/Ir von 84/16, eine Flächendichte des Edelmetalls von 6 g/m², in einer zweiten Variante, entsprechend einem Verhältnis Ru/Ir von 70/30, eine Flächendichte des Edelmetalls von 7,5 g/m², in einer dritten Variante, entsprechend einem Verhältnis Ru/Ir von 50/50, eine Flächendichte von 9 g/m², für eine Beschichtung des Typs "Pt-IrO₂", entsprechend einem Verhältnis Pt/Ir von 70/30, eine Flächendichte des Edelmetalls von 10 g/m² und für eine Beschichtung des Typs "Pt-RU-Mischoxid", entsprechend einem Verhältnis Pt/Ru von 50/50, eine Flächendichte des Edelmetalls von 11 g/m² vorgesehen.

Vorzugsweise wird die Elektrolysezelle der genannten Art in Rahmen einer Anlage zur Erzeugung einer elektrochemisch aktivierten Lösung durch Elektrolyse von solehaltigem Wasser eingesetzt.

Die genannte, auf die Anlage zur Erzeugung der elektrochemisch aktivierten Lösung gerichtete Aufgabe wird gelöst mit einem eine Mehrzahl von Elektrolysezellen der genannten Art umfassendes Elektrolysemodul, bei dem die Anodenräume der Elektrolysezellen medienseitig in Reihe geschaltet sind, wobei die Oberflächenbeschichtung, insbesondere hinsichtlich ihrer Materialbestandteile, der Anode der jeweiligen Elektrolysezelle jeweils abhängig von deren Position in der Reihenschaltung gewählt ist.

Wie sich nämlich überraschend herausgestellt hat, ist gerade durch die medienseitige Hintereinanderschaltung einer Mehrzahl von Anodenräumen der genannten Art eine besonders zielgerichtete und weitgehende Aufkonzentration des Gehalts an freiem Chlor in der elektrochemisch aktivierten Lösung auch bei hoher Lagerbeständigkeit des Produkts auf besonders günstige Weise erreichbar. Gerade durch die Hintereinanderschaltung einer Mehrzahl von Anodenräumen der genannten Art ist dabei innerhalb des Medienstroms eine besondere gezielte und an die jeweilige Stufe des Herstellungsprozesses angepasste Behandlung des Elektrolyten in den Anodenräumen ermöglicht. Dabei kann in besonderem Maße durch eine geeignete, von der Position des jeweiligen Anodenraums innerhalb der Reihenschaltung der Anodenräume abhängig gewählte Beschichtung eine besonders gezielte lokale Modifikation des Elektrolyten im jeweiligen Anodenraum erreicht werden. Durch eine geeignete, abhängig von der Position des jeweiligen Anodenraums in der Hintereinanderschaltung gewählte Wahl der Bestandteile der Beschichtung oder ihres Mischungsverhältnisse zueinander können somit verschiedene Herstellungsparameter besonders aufeinander abgestimmt werden, so dass eine besonders gezielte Aufkonzentration des freien Aktivchlorgehalts entlang des Strömungswegs des Mediums durch die Kaskadenschaltung der Anodenräume und auch eine besonders günstige Beeinflussung der Lagerbeständigkeit erreicht werden können.

In besonders bevorzugter Weiterbildung sind dabei eine oder einige der Elektrolysezellen nach der vorstehend genannten Art ausgeführt, umfassen also auf ihrer Anode eine Oberflächenbeschichtung mit einem geeignet gewählten Anteil an Rutheniumoxid.

Insbesondere in der oder den medienstromseitig vergleichsweise weit vorne angeordneten Elektrolysezellen ist dabei die Oberflächenbeschichtung der Anode besonders vorteilhaft an eine vergleichsweise hohe Produktionsrate Sauerstoff-abspaltender Verbindungen, also insbesondere eine hohe Cl-Produktionsrate, angepasst, wohingegen in der oder den vergleichsweise hinten angeordneten Elektrolysezellen die Cl-Produktionsrate eher geringer gehalten werden kann. In besonders vorteilhafter Ausgestaltung ist somit die Oberflächenbeschichtung der Anode einer in der medienseitigen Reihenschaltung gesehen vergleichsweise hinten angeordnete Elektrolysezelle mit einem vergleichsweise geringeren Anteil an Rutheniumoxid und/oder Iridiumoxid versehen als die Oberflächenbeschichtung der Anode einer in der medienseitigen Reihenschaltung gesehen vergleichsweise vorne angeordneten Elektrolysezelle.

Bei dieser Auslegung ist in besonderem Maße der überraschenden Erkenntnis Rechnung getragen, dass für die allgemeinen Eigenschaften der hergestellten elektrochemisch aktivierten Salzlösung, insbesondere im Hinblick auf Lagerbeständigkeit, Verträglichkeit mit weiteren Stoffen und dergleichen, diverse während des Elektrolyseprozesses anfallende Nebenprodukte besonders schädlich zu sein scheinen. Wie sich überraschenderweise herausgestellt hat, ist die Produktionsrate derartiger Nebenprodukte in den einzelnen Elektrolysezellen dann besonders gering, wenn das Produkt oder der Elektrolyt in diesen Elektrolysezellen alkalisch ist, also einen pH-Wert von mehr als beispielsweise 7,5 aufweist. Eine elektrochemisch aktivierte Salzlösung mit besonders günstigen Eigenschaften ist also vorzugsweise herstellbar, indem in weiten Teilen der Herstellungsprozess in alkalischer Umgebung vorgenommen wird. Dies ist dadurch begünstigt, dass der Schwerpunkt der Erzeugung Sauerstoff-abspaltender Verbindungen, also insbesondere der Cl-Erzeugung, erreichbar durch einen geeignet hohen Ru-Anteil in der Oberflächenbeschichtung der jeweiligen Anode, in die vergleichsweise vorne angeordneten Elektrolysezellen verlagert wird.

Um dabei dennoch letzten Endes ein pH-neutrales Produkt herstellen zu können, wird vorteilhafterweise bei der Herstellung der elektrochemisch aktivierten Salzlösung in der kaskadenartig aufgebauten Reihenschaltung der genannten Art in der letzten Stufe, also in der medienseitigen Reihenschaltung gesehen letzten Elektrolysezelle, die alkalische Umgebung des Elektrolyten aufgehoben und der pH-Wert auf einen Wert von etwa 7,3 abgesenkt. In dieser letzten Stufe ist somit vermehrt mit dem Anfall der genannten Nebenprodukte zu rechnen. Um dem Rechnung zu tragen, wird in vorteilhafter Weise durch eine geeignete Wahl der Oberflächenbeschichtung der Anode gerade in dieser medienseitig gesehen letzten Elektrolysezelle die Cl-Produktionsrate besonderes gering gehalten, so dass trotz des dort vorgesehenen Absenkens des pH-Werts auf einen neutralen Bereich der Anfall der eigentlich unerwünschten, die Eigenschaften des Produktes insgesamt unerwünscht beeinflussenden Nebenprodukte gerade aufgrund der geringen Cl-Produktionsrate besonders gering gehalten werden kann. Um diesen Randbedingungen geeignet Rechnung zu tragen, ist in besonderes vorteilhafter Ausgestaltung die Oberflächenbeschichtung der Anode der in der medienseitigen Reihenschaltung gesehen letzten Elektrolysezelle im Wesentlichen Rutheniumoxid-frei aufgeführt. Um dem gegenüber aber auch die zur gewünschten Herabsetzung des pH-Werts auf neutrale Werte geeigneten elektrochemischen Vorgänge in der in Medienstromrichtung gesehen letzten Elektrolysezelle besonders geeignet zu begünstigen, ist die Oberflächenbeschichtung der Anode dieser Elektrolysezelle in weiterer vorteilhafter Ausgestaltung im Wesentlichen auf Platin-Basis ausgeführt.

Alternativ oder zusätzlich kann dabei in einer oder mehreren der Elektrolysezellen die Oberflächenbeschichtung der Anode sowohl Ruthenium oder Rutheniumoxid als auch Platin oder Platinoxid in der Art einer Reischung dieser Bestandteile enthalten sein. Das Mischungsverhältnis der Ruthenium-basierten Bestandteile zu den Platin-basierten Bestandteilen kann dabei Anteilhalterweise ebenfalls positionsabhängig, als abhängig von der Position der jeweiligen Elektrolysezelle innerhalb der Kaskadenschaltung, gewählt sein, wobei insbesondere der Rutheniumbasierte Anteil in der Oberflächenbeschichtung "nach hinten hin" innerhalb der Serienschaltung abnimmt.

Bezüglich des Verfahrens zur Erzeugung einer elektrochemisch aktivierten Lösung durch Elektrolyse von solehaltigem Wasser wird die genannte Aufgabe gelöst, indem eine Anlage der genannten Art, also insbesondere umfassend eine Mehrzahl von mit ihren Anodenräumen medienseitig in Reihe geschalteten Elektrolysezellen, verwendet wird, wobei der Elektrolyt den Kathodenräumen der Elektrolysezellen parallel und den Anodenräumen der Elektrolysezellen in Reihenschaltung zugeführt wird. Wie sich nämlich überraschenderweise herausgestellt hat, kann gerade auf diese Weise im Anolyten nach dem Durchlaufen von mehreren, insbesondere von mindestens zwei, Behandlungsstufen ein besonderes hoher Gehalt von freiem Chlor von bis zu 2.000 mg/l erreicht werden, wobei der solchermaßen hergestellte Anolyt zudem auch noch eine besonders hohe Lagerbeständigkeit aufweist.

Besonders günstige Materialeigenschaften des Anolyts hinsichtlich des Gehalts an freiem Chlor und auch hinsichtlich der Lagerbeständigkeit sind zudem erreichbar, indem in vorteilhafter Weiterbildung die Kathodenräume der Elektrolysezellen medienabströmseitig mit dem Anodenraum der in Strömungsrichtung des Anolyten gesehen ersten Elektrolysezelle verbunden sind. Bei dieser besonders bevorzugten medienseitigen Schaltung wird der aus den Kathodenräumen der Elektrolysezellen abströmende Elektrolyt - vorzugsweise ein daraus geeignet abgezweigter Teilstrom - dem Anodenraum der in Strömungsrichtung des Elektrolyten gesehen ersten Elektrolysezelle zugeführt.

Die mit der Erfindung erzeilten Vorteile bestehen insbesondere darin, dass durch die geeignete Wahl der Oberflächenbeschichtung der Anode, nämlich insbesondere durch die Bereitstellung von Rutheniumoxid und/oder Iridiumoxid als Bestandteil der Oberflächenbeschichtung, gezielt die Cl-Produktionsrate und/oder ggf. die O₂-Produktionsrate begünstigt werden kann, wobei zudem die geeignete Bereitstellung von Iridiumoxid im hergestellten Produkt auch noch eine besonders hohe Lagerbeständigkeit begünstigt. Gerade durch eine positions- oder ortsabhängig angepasste Wahl der Beschichtung und der sie bildenden Komponenten innerhalb der Reihenschaltung einer Mehrzahl von Elektrolysezellen ist zudem sichergestellt, dass eine besonders bedarfsangepasste Beeinflussung der elektrochemischen Vorgängen während der Elektrolyse erreicht werden kann.

Insbesondere durch die innerhalb der Kaskadenschaltung der Anodenräume der Elektrolysezellen abnehmende Cl-Produktion infolge gezielt reduzierten Rutheniumoxid-Anteils in der Oberflächenbeschichtung kann dabei die Aufkonzentration des freien Chlors im Medium gezielt an die Strömungsverhältnisse bei der Durchströmung der Kaskadenschaltung der Anodenräume angepasst und damit besondes effizient ausgestaltet werden. Durch die Bereitstellung einer im wesentlichen Rutheniumoxid-freien Oberflächenbeschichtung im Anodenraum der Medienstromseitig gesehen letzten Elektrolysezelle kann zudem die Anpassung des pH-Werts des hergestellten Produkts an einen neutralen Bereich und damit die Kompatibilität des Produkts mit einer Vielzahl anderer Substanzen oder Wirkstoffe gezielt hergestellt werden, ohne dass hierdurch eine erhöhte Erzeugungsrate an sich unerwünschter und die Eigenschaften des Produkts negativ beeinflussender Nebenprodukte in Kauf genommen werden müsste. Durch die genannten Ausführungsformen der Oberflächenbeschichtung kann somit eine elektrochemisch aktivierte Lösung hergestellt werden, die bei hoher Lagerbeständigkeit einen besonders günstigen pH-Wert und einen besonders hohen Gehalt an freiem Chlor bei vergleichsweise hohem Redox-Potential aufweist.

Ein Ausführungsbeispiel der Erfindung wird anhand einer Zeichnung näher erläutert. Darin zeigen:
- FIG. 1: eine Anlage zur Erzeugung einer elektrochemisch aktivierten Salzlösung durch Elektrolyse, und
- FIG. 2: eine Anodenkammer der Anlage nach Fig. 1 im Längsschnitt.
Gleiche Teile sind in beiden Figuren mit denselben Bezugszeichen versehen.

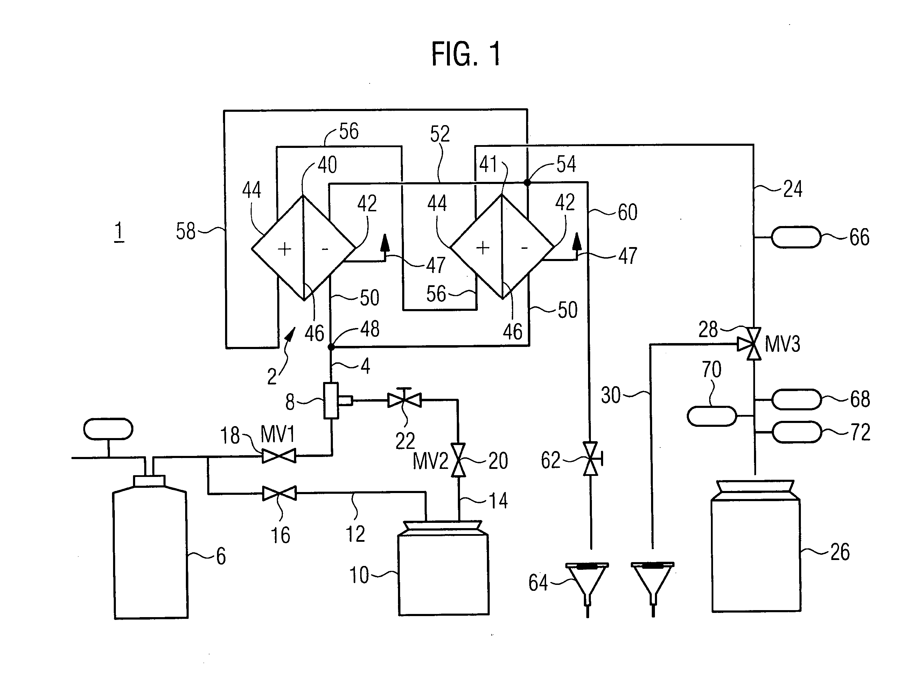

Die Anlage 1 gemäß Figur 1 ist zur Erzeugung einer elektrochemisch aktivierten Salzlösung durch Elektrolyse vorgesehen. Dazu umfasst die Anlage 1 ein Elektrolysemodul 2, dem eingangsseitig über eine Versorgungsleitung 4 ein Elektrolysemedium zuführbar ist. Als Elektrolysemedium ist dabei mit Sole oder einer wässrigen Salzlösung versetztes enthärtetes Wasser vorgesehen. Dazu ist die Zuströmleitung 4 eingangsseitig mit einer Wasserenthärterstation 6 verbunden. Zur Zudosierung der Sole in das enthärtete Wasser ist in die Zuströmleitung 4 eine Venturidüse 8 geschaltet, die ihrerseits eingangsseitig mit einem Solebehälter 10 verbunden ist. Von der Zuführungsleitung 4 zweigt zudem nach der Wasserenthärtestation 6 eine Ablaufleitung 12 ab, über die während einer Inbetriebnahmephase der Anlage 1 der Wasserstrom aus der Wasserenthärterstation 6 unter Umgehung des Elektrolysemoduls 2 der nachfolgenden Komponenten in den Solebehälter 10 abführbar ist. Zur Umschaltung zwischen diesen Betriebszuständen mit dem Dauerbetriebszustand sowie zur dosierten Einspeisung einer vorgebbaren Solemenge in das Elektrolysemedium sind in die Zuführleitung 4, die Ablaufleitung 12 sowie die in die Venturidüse 8 mündende Soleeinspeiseleitung 14 geeignete Ventile 16, 18, 20 und zusätzlich in die Einspeiseleitung 14 ein Drosselventil 20 geschaltet.

Ausgangsseitig, zur Abführung der im Elektrolysemodul 2 hergestellten elektrochemisch aktivierten Salzlösung, ist an das Elektrolysemodul 2 eine Abströmleitung 24 angeschlossen, die ausgangsseitig in einen Vorratsbehälter 26 für die hergestellte Salzlösung oder den Anolyten mündet. Zur vorübergehenden Umgehung des Vorratsbehälters 26 während der Inbetriebnahmephase der Anlage 1 ist in die Ablaufleitung 24 zudem ein Multiwegeventil 28 geschaltet, dessen zweiter Ausgang an eine Abflussleitung 30 angeschlossen ist.

Die Anlage 1 ist zur Herstellung einer elektrochemisch aktivierten Salzlösung mit gerade zur Verwendung als Desinfektionsmittel oder antibakteriellem Wirkstoff in beispielsweise medizinischen oder pharmazeutischen Anwendungen besonders günstigen Eigenschaften, insbesondere mit einem besonders hohen Gehalt an freiem Chlor von vorzugsweise mehr als 500 mg/l und insbesondere sogar mehr als 2.000 mg/l bei hoher Lagerfähigkeit, ausgelegt. Um dies zu ermöglichen, ist das Elektrolysemodul 2 mehrkomponentig aufgebaut und umfasst eine Mehrzahl von Elektrolysezellen 40, 41, von denen im Ausführungsbeispiel lediglich zwei dargestellt sind; selbstverständlich können analog zu den folgenden Ausführungen aber auch noch weitere Elektrolysezellen 40, 41 vorgesehen sein.

Jede Elektrolysezelle 40, 41 umfasst jeweils einen einen ersten Elektrodenraum bildenden Kathodenraum 42 und einen einen zweiten Elektrodenraum bildenden Anodenraum 44, die jeweils durch eine Membran 46 voneinander getrennt sind. Das Anlegen einer elektrischen Spannung zwischen einer den jeweiligen Anodenraum 44 begrenzenden Anode einerseits und einen den jeweiligen Kathodenraum 42 begrenzenden Kathode andererseits bewirkt sodann eine Ionenwanderung über die zwischen liegende Membran 46 hinweg, so dass eine zumindest teilweise Dissoziation des im Elektrolysemedium enthaltenen Wassers sowie eine zumindest teilweise Dissoziation der in Form von Sole mitgeführten Salzanteile auftritt. Auf Grund der elektrischen Ladung der Ionen in diesen Materialien führt dieser Ionenwanderungsprozess in Folge der angelegten elektrischen Spannung zu einer Anreicherung von Cl⁻ und OH- im jeweiligen Anodenraum 44 und zu einer Anreicherung von H⁺ und Na⁺ im jeweiligen Kathodenraum 42. Zur bedarfsweisen Abführung von demzufolge im jeweiligen Kathodenraum 42 oder ersten Elektrodenraum angereichertem Wasserstoffgas ist der Kathodenraum 42 jeweils mit einer Entgasungsleitung 47 verbunden. Diese bildet, ggf. in Kombination mit im Kathodenraum 42 angeordneten Mitteln zur Bläschenerzeugung oder Gasabscheidung wie beispielsweise Verwirbler oder dergleichen, ein Entgasungsmodul für den jeweiligen Kathodenraum 42.

Die gewünschten, qualitativ hochwertigen Eigenschaften der elektrochemisch aktivierten Salzlösung werden in der Anlage 1 insbesondere durch eine spezifische Führung der Medienströme im Elektrolysemodul 2 erreicht. Dabei ist in der Anlage 1 gemäß dem Ausführungsbeispiel, die gezielt auf die Bereitstellung von elektrochemisch hochgradig aktiviertem Anolyt ausgerichtet ist, eine kathodenseitig im Wesentlichen parallele Medienstromführung mit einer anodenseitig im Wesentlichen seriellen Medienstromführung kombiniert. Alternativ oder bei abweichendem Auslegungsziel kann auch eine anodenseitig im Wesentlichen parallele mit einer kathodenseitig im Wesentlichen seriellen Medienstromführung kombiniert sein. Darüberhinaus können auch noch weiterführende Kombinationen dieser medienseitigen Verschaltungen vorgesehen sein.

Im Einzelnen ist dabei in der Anlage 1 im Ausführungsbeispiel in der Zuströmleitung 4 ein Verzweigungspunkt 48 vorgesehen, von dem aus in der Art einer parailelen Medienzuführung in die Kathodenräume 42 der Elektrolysezellen 40, 41 mündende Zuleitungen 50 abgehen. Ausgangsseitig der Kathodenräume 42 sind Abströmleitungen 52 vorgesehen, die in einem Sammelpunkt 54 zusammengeführt sind, so dass sich insgesamt eine medienseitig parallele Verschaltung der Kathodenräume 42 ergibt.

Anodenseitig ist hingegen eine Reihen- oder Hintereinanderschaltung der Anodenräume 44 für den Anolyten vorgesehen. Dazu ist der in Strömungsrichtung des Anolyten gesehen dem ersten Elektrolysemodul 40 zugeordnete Anodenraum 44 ausgangsseitig über eine Überströmleitung 56 mit der Eingangsseite des nachfolgenden, im zweiten Elektrolysemodul 41 angeordneten Anodenraums 44 verbunden. Dieser ist seinerseits ausgangsseitig an die Abströmleitung 24 angeschlossen, so dass sich in der Art einer mehrstufigen oder kaskadenartigen Ausführung eine Hintereinanderschaltung der Anodenräume 44 bzgl. des Anolyten ergibt. Zudem ist in der Anlage 1 vorgesehen, den aus den Kathodenräumen 42 abströmenden Katholyten als Anolyt in die hintereinander geschalteten Anodenräume 44 einzuspeisen. Dazu ist der Sammelpunkt 54 für den Katholyten über eine Überströmleitung 58 mit dem Anodenraum 44 der in Strömungsrichtung des Anolyten gesehen ersten Elektrolysezelle 40 verbunden.

Von der Überströmleitung 58 zweigt im Sammelpunkt 54 zudem eine Abführleitung 60 ab, über die ein in seiner Menge über ein Drosselventil 62 einstellbarer Teilstrom des Katholyten einem Abwassersystem oder einem Auffangbehälter 64 zugeführt werden kann. Mit dieser Anordnung ist es möglich, eine einstellbare Teilmenge des aus den Kathodenräumen 42 abströmenden Katholyten als Anolyt der Kaskade aus Anodenräumen 44 zuzuführen. Damit können unter anderem die individuellen Reaktionsparameter wie beispielsweise Druck und Fließgeschwindigkeit in den Anodenräumen 44 geeignet beeinflusst werden, und zudem kann die Menge des in diesem Anwendungsfall als "Abfallprodukt" anfallenden Katholyts besonders gering gehalten werden.

Zur Überprüfung der Materialeigenschaften des hergestellten Anolyten sowie zur Mengenmessung sind im Übrigen in die Abströmleitung 24 eine Anzahl von Sensoren, insbesondere ein Mengensensor 66, ein Temperatursensor 68, ein pH-Sensor 70 sowie ein Sensor 72 zur Messung des Redoxpotentials, geschaltet.

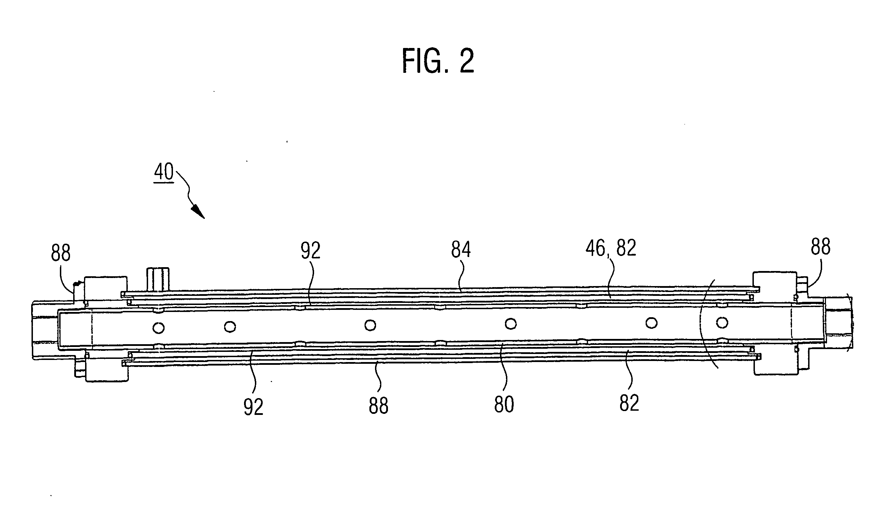

In konstruktiver Hinsicht sind die Elektrolysezellen 40, 41, wie dies im Längsschnitt in Figur 2 gezeigt ist, in im Wesentlichen zylindrischer Grundform ausgeführt. Dabei ist ein zur Bildung der Anode vorgesehener, als Hohlzylinder ausgeführter Grund- oder Trägerkörper 80 konzentrisch von einem die Membran 46 bildenden, ebenfalls hohlzylindrisch ausgeführten Keramikkörper 82 umgeben. Zur Bildung des Kathodenraums 42 ist der Keramikkörper 82 seinerseits konzentrisch von einem hohlzylindrischen Außenkörper 84 umgeben. Endseitig sind diese Komponenten über geeignete Abschlusselemente 88 geeignet verschlossen und gegebenenfalls abgedichtet, wobei über Medienzuströmöffnungen 90 die Zuführung des Elekrolyten in den die Anode bildenden Grund- oder Trägerkörper 80 erfolgen kann.

Um die beim Elektrolysevorgang auftretenden elekrochemischen Prozesse in den Elektrolysezellen 40, 41 im besonderem Maße hinsichtlich der angestrebten Ergebnisse, nämlich insbesondere einer besonders bedarfgerecht gesteigerten Cl-Produktion, zu begünstigen, ist der zur Bildung der Anode vorgesehene Grund-oder Trägerkörper 80 auf seiner Oberfläche mit einer spezifisch gewählten Beschichtung versehen. Diese Beschichtung 92 ist hinsichtlich ihrer Materialwahl und -zusammensetzung individuell an die Erfordernisse im Elektrolyseprozess angepasst und insbesondere abhängig von der Position der jeweiligen Elektrolysezelle 40, 41 innerhalb der kaskadenartigen medienstromseitigen Reihenschaltung der Anodenräume 44 gewählt.

Für den in Medienstromrichtung gesehen innerhalb der kaskadenartigen Reihenschaltung ersten Anodenraum 44 ist die auf der Anode vorgesehene Oberflächen-beschichtung 92 dabei in besonderem Maße für eine besonders hohe Produktionsrate an freiem Chlor ausgelegt. Zu diesem Zweck umfasst die Oberflächenbeschichtung 92 der Anode in der Elektrolysezelle 40 Rutheniumoxid und weitere Bestandteile, insbesondere von Iridiumoxid, im Ausführungsbeispiel in einem Mischungsverhältnis von etwa 70/30 (Ru/Ir), bezogen auf die Mengenanteile der bei Herstellung der Oberflächenbeschichtung verwendeten Ausgangspaste.

Im Gegensatz dazu ist die Anode in der medienstromseitig gesehen letzten Elektroysezelle 41 mit einer Oberflächenbeschichtung versehen, die im Wesentlichen Rutheniumoxid-frei ausgeführt ist. Dadurch ist gewährleistet, dass in der in Medienstromrichtung gesehen letzten Elektrolyezelle 41 die Produktionsrate an freiem Chlor vergleichsweise gering gehalten ist, so dass in dieser Elektrolysezelle 41 der pH-Wert des Anolyten auf einen für eine gute Kompartibilität mit sonstigen Substanzen oder Wirkstoffen geeigneten vergleichsweise neutralen Wert herabgesetzt werden kann, ohne dass hierdurch bedingt mit der an sich unerwünschten Produktion von schädlichen Nebenprodukten gerechnet werden müsste. Zu diesem Zweck ist im Ausführungsbeispiel die Oberflächenbeschichtung 92 der Anode in der Elektrolysezelle 41 im Wesentlichen auf Platin-Basis ausgeführt. Selbstverständlich können im Elektrolysemodul 2 noch weitere Elektrolysezellen innerhalb der kaskadenartigen Serienschaltung der Anodenräume vorgesehen sein; in diesem Fall ist die Oberflächenbeschichtung der jeweiligen Anode entsprechend den positionsabhängig spezifierten Bedürfnissen bei gezielter Beeinflussung der elektrochemischen Vorgänge während der Elektrolyse geeignet zu wählen.

### Bezugszeichenliste

- 1: Anlage
- 2: Elektrolysemodul
- 4: Versorgungsleitung
- 6: Wasserenthärterstation
- 8: Venturidüse
- 10: Solebehälter
- 12: Ablaufleitung
- 14: Soleeinspeiseleitung
- 16,18,20: Ventil
- 20: Drosselventil
- 24: Abströmleitung
- 26: Vorratsbehälter
- 28: Multiwegeventil
- 30: Abflussleitung
- 40,41: Elektrolysezellen
- 42: Kathodenraum
- 44: Anodenraum
- 46: Membran
- 47: Entgasungsleitung
- 48: Verzweigungspunkt
- 50: Zuleitungen
- 52: Abströmleitungen
- 54: Sammelpunkt
- 56: Überströmleitung
- 60: Abführleitung
- 62: Drosselventil
- 64: Auffangbehälter
- 66: Mengensensor
- 68: Temperatursensor
- 70: pH-Sensor
- 72: Sensor
- 80: Grundkörper
- 82: Keramikkörper
- 84: Außenkörper
- 90: Medienzuströmöffnungen

## Patentansprüche

1. Elektrolysezelle (40), insbesondere zur Erzeugung einer elektrochemisch aktivierten Kochsalzlösung, mit einem mit einer Anode versehenen Anodenraum (44) und mit einem von diesem durch eine Membran (46) getrennten, mit einer Kathode versehenen Kathodenraum (42), **dadurch gekennzeichnet, dass** die Anode auf ihrer Oberfläche eine Beschichtung (92) umfassend Rutheniumoxid und Iridiumoxid aufweist.

2. Elektrolysezelle (40) nach Anspruch 1, bei der die Oberflächenbeschichtung (92) erhalten ist durch Bestreichen eines Trägerkörpers (80) mit einer die Metallanteile enthaltenden Paste und anschließendes Ausbrennen und Oxidieren.

3. Elektrolysezelle (40) nach Anspruch 1 oder 2, bei der die Bestandteile der Oberflächenbeschichtung (92) der Anode in einem Verhältnis Rutheniumoxid/Iridiumoxid, insbesondere der Gewichtsanteile in der zum Bestreichen des Trägerkörpers vorgesehenen Ausgangspaste, von zwischen 50/50 (Ru/Ir) und 95/5 (Ru/Ir) (man könnte auch schreiben 100% Ru, Kaehn sagt, dass 95/5 sonst keiner macht - auch bei 95/5 ist kaum Ir an der Oberfläche, sondern eher nur Verbindungsschicht, Kleber. Gaas sagt, es gibt kein reines RuOxid), vorzugsweise von etwa 70/30 (Ru/Ir), vorhanden sind.

4. Elektrolysezelle (40) nach einem der Ansprüche 1 bis 3, bei der die Oberflächenbeschichtung (92) eine Flächendichte des Edelmetallanteils von etwa 5 g/m² bis etwa 11 g/m² aufweist.

5. Anlage (1) zur Erzeugung einer elektrochemisch aktivierten Lösung mit einem Elektrolysemodul (2), das eine eine Mehrzahl von Elektrolysezellen (40, 41), von denen zumindest eine insbesondere nach einem der Ansprüche 1 bis 4 ausgestaltet ist, umfasst, und bei dem die Anodenräume (44) der Elektrolysezellen (40, 41) medienseitig in Reihe geschaltet sind, wobei die Oberflächenbeschichtung (92) der Anode jeder Elektrolysezelle (40, 41) jeweils abhängig von der Position der jeweiligen Elektrolysezelle in der Reihenschaltung gewählt ist.

6. Anlage (1) nach Anspruch 5, bei der die Oberflächenbeschichtung (92) der Anode einer in der medienseitigen Reihenschaltung gesehen vergleichsweise hinten angeordneten Elektrolysezelle (41) einen vergleichsweise geringeren Anteil an Rutheniumoxid und/oder Iridiumoxid aufweist als die Oberflächenbeschichtung der Anode einer in der medienseitigen Reihenschaltung gesehen vergleichsweise vorne angeordneten Elektrolysezelle (40).

7. Anlage (1) nach Anspruch 5 oder 6, bei der die Oberflächenbeschichtung (92) der Anode der in der medienseitigen Reihenschaltung gesehen letzten Elektrolysezelle (41) im wesentlichen Rutheniumoxid- und/oder Iridiumoxid-frei ausgeführt ist.

8. Verfahren zur Erzeugung einer elektrochemisch aktivierten Lösung durch Elektrolyse von solehaltigem Wasser, bei dem eine Anlage (1) nach einem der Ansprüche 5 bis 7 verwendet wird, wobei der Elektrolyt den Kathodenräumen (42) der Elektrolysezellen (40, 41) parallel und den Anodenräumen (44) der Elektrolysezellen (40, 41) in Reihenschaltung zugeführt wird.

9. Verfahren nach Anspruch 8, bei dem aus den Kathodenräumen (42) der Elektrolysezellen (40, 41) abströmender Elektrolyt dem Anodenraum (44) der in Strömungsrichtung des Elektrolyten gesehen ersten Elektrolysezelle (40) zugeführt wird.

10. Verfahren nach Anspruch 9, bei dem lediglich ein Teilstrom des aus den Kathodenräumen der Elektrolysezellen (40, 41) abströmenden Elektrolyts dem Anodenraum (44) der in Strömungsrichtung des Elektrolyten gesehen ersten Elektrolysezelle (40) zugeführt wird.
